(19) Europäisches Patentamt
European Patent Office
Office européen des brevets

(11) Veröffentlichungsnummer: **0 134 041**
**B1**

(12) # EUROPÄISCHE PATENTSCHRIFT

(45) Veröffentlichungstag der Patentschrift: **06.04.88**

(21) Anmeldenummer: **84110072.0**

(22) Anmeldetag: **23.08.84**

(51) Int. Cl.⁴: **C 07 D 209/76**, C 07 C 125/06, C 08 B 3/00, C 07 K 17/06, C 12 N 11/06

(54) **N-Chlorcarbonyloxy-5-norbornen-2,3-dicarboximid, Verfahren zu seiner Herstellung und seine Verwendung.**

(30) Priorität: **25.08.83 DD 254196**
**25.08.83 DD 254197**
**25.08.83 DD 254198**
**28.10.83 DD 256056**
**28.10.83 DD 256057**

(43) Veröffentlichungstag der Anmeldung: **13.03.85 Patentblatt 85/11**

(45) Bekanntmachung des Hinweises auf die Patenterteilung: **06.04.88 Patentblatt 88/14**

(84) Benannte Vertragsstaaten: **AT BE CH DE FR GB IT LI NL SE**

(56) Entgegenhaltungen:
**DE-A-3 131 250**

**CANADIAN JOURNAL OF CHEMISTRY, Band 60, Nr. 8, 15. April 1982, A. PAQUET "Introduction of 9-fluorenylmethyloxycarbonyl, trichloroethoxycarbonyl, and benzyloxycarbonyl amine protecting groups into O-unprotected hydroxyamino acids using succinimidyl carbonates", Seiten 976-980**
**BIOTECHNOLOGY AND BIOENGINEERING, Band 24, 1982, JOHN WILEY & SONS, INC., J. DROBNIK et al. "The Activation of Hydroxy Groupgs of Carriers with 4-Nitrophenyl and N-Hydroxysuccinimidyl Chloroformates", Seiten 487-492**

(73) Patentinhaber: **Akademie der Wissenschaften der DDR, Otto- Nuschke- Strasse 22/23, DDR- 1086 Berlin (DD)**

(72) Erfinder: **Henklein, Peter, Dr. Dipl.- Chem., Schulze- Boysen- Strasse 25, DDR- 1130 Berlin (DD)**
Erfinder: **Becker, Manfred, Dr. Dipl.- Biol., Mendelstrasse 52, DDR- 1100 Berlin (DD)**
Erfinder: **Büttner, Werner, Dr. Dipl.- Chem., Trelleborger Strasse 41, DDR- 1100 Berlin (DD)**
Erfinder: **Loth, Fritz, Dr. Dipl.- Chem., Ernst-Thälmann- Strasse 211, DDR- 1530 Teltow (DD)**
Erfinder: **Dautzenberg, Horst, Dr. Dipl.- Chem., Moldaustrasse 2 b, DDR- 1530 Teltow (DD)**
Erfinder: **Forner, Klaus, Dr. Dipl.- Chem., Florastrasse 34, DDR- 1100 Berlin (DD)**
Erfinder: **Dölling, Rudolf, Dr. Dipl.- Chem., Lieselotte- Herrmann- Strasse 7, DDR- 1055 Berlin (DD)**
Erfinder: **Graul, Karl- Heinz, Dipl.- Chem., Clara-Zetkin- Strasse 22, DDR- 4300 Quedlinburg (DD)**
Erfinder: **Halatsch, Wolf- Rainer, Dr. Dipl.- Chem., Moskauer Strasse 34/35, DDR- 1405 Glienicke (DD)**
Erfinder: **Rupprich, Christian, Dipl.- Ing., Langhoffstrasse 26, DDR- 1146 Berlin (DD)**

(74) Vertreter: **Patentanwälte Beetz sen. - Beetz jun. Timpe - Siegfried - Schmitt- Fumian, Steinsdorfstrasse 10, D-8000 München 22 (DE)**

**EP 0 134 041 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft die neue Verbindung N-Chlor-carbonyloxy-5-norbornen-2,3-dicarboximid, Verfahren zu ihrer Herstellung sowie ihre Verwendung. Diese Verbindung kann vorteilhaft als Zwischenprodukt zur Herstellung unsymmetrischer Carbonate und aktivierter polymerer Träger verwendet werden. Die Erfindung ist entsprechend insbesondere in der pharmazeutischen und in der chemischen Industrie sowie der Biotechnologie anwendbar.

Chlorameisensäureester sind als Verbindungen von großer präparativer Bedeutung bekannt. Für ihre Herstellung aus Phosgen und Hydroxylverbindungen werden drei Verfahren angewandt: Beim wichtigsten Verfahren wird Phosgen direkt mit den Alkoholen umgesetzt [B. Röge, Liebigs Ann. Chem. 205, 227 (1880); Houben-Weyl, 4. Aufl. (1952), Bd. 8, S. 101 bis 102; US-A 2 476 637]. Dazu wird entweder flüssiges Phosgen oder eine Phosgenlösung in einem inerten organischen Lösungsmittel (Toluol) vorgelegt und der Alkohol zugetropft [W. Hentschel, Chem. Bericht 18, 1177 (1885)] oder umgekehrt Phosgen durch den gekühlten Alkohol geleitet [C. Hamilton, C. Sly, Amer. Chem. Soc. 47, 436 (1925)].

Anderen Vorschriften gemäß arbeitet man mit tertiären Aminen, die den bei der Reaktion entstehenden Chlorwasserstoff abfangen sollen. Als tertiäre Amine werden z. B. Dimethylanilin oder Pyridin verwendet. Dieses Verfahren eignet sich vor allem zur Herstellung aromatischer Chlorameisensäureester. In anderen Vorschriften zur Herstellung von aromatischen Chlorameisensäureestern werden Alkaliphenolate zur Chlorwasserstoffbindung bei der Umsetzung mit Phosgen genannt. Diesen Beschreibungen zufolge wird in inerten Lösungsmitteln [K. v. Anwers, W. Scheich, Chem. Ber. 54, 1969 (1921)] oder auch in wäßrigorganischen Lösungsmittelsystemen gearbeitet [E. Barrat, A. Morel, Comptes Rendus Hebdomadaires des Séances de l'Académie des Sciences 128, 1578 (1899)].

Bedeutung haben die Chlorameisensäureester des 9-Fluorenyl-methanols, des Pentachlorphenols, des Benzotriazols und des N-Hydroxysuccinimids als Vorprodukte für Schutzgruppen in der Peptidsynthese [A. Paquet, Can. J. Chem. 60, 976 (1982)]. Diese Chlorameisensäureester sind aber, bedingt durch ihre geringe Hydrolysestabilität oder zu geringe Aminolysegeschwindigkeit, nicht vorteilhaft anwendbar.

Ferner ist eine Vielzahl symmetrischer Carbonate bekannt, die zur Einführung von Urethan-Schutzgruppen in Aminosäuren verwendet werden. Die t-Butyloxycarbonyl-(Boc-) Gruppe stellt neben der Benzyloxycarbonyl-(Z-)-Gruppe die in der Peptidsynthese am häufigsten benutzte Schutzgruppe dar. Um die in der hohen Toxizität und Explosionsneigung bestehenden Nachteile des bislang meistverwendeten Boc-Einführungsreagens, des t-Butyloxycarbonylazids [Schwyzer, R., Sieber, P., u. Kappler, H. Helv. Chim. Acta 42, 2622 (1962)] zu vermeiden, wurden neue Einführungsreagentien in großer Zahl entwickelt, wie t-Butyl-4,6-dimethylpyrimidyl-2-thiolcarbonat [Nagasawa, T., Kuriowa, K., Narita, K., u. Isowa, Y. Bull. Chem. Soc. (Jap.) 46, 1269 (1973), und Mc Gregor, A. C., J. Am. Chem. Soc. 79, 6180 (1957)], t-Butyloxycarbonyloxyimino-2-phenylacetonitril [Itoch, M., Hagiwara, D., u. Kamiya, T., Bull. Chem. Soc. (Jap.) 50, 718 (1977); Tetrahedron Lett. 1975, 4393], N-t-Butyloxycarbonyloxy)-phthalimid [Gross, H., u. Bilk, L., Liebigs Ann. Chem. 725, 212 (1969)], N-t-Butyloxycarbonyloxy)-succinimid [Franket, M., Ladkany, D., Gilon C., u. Wolman, Y., Tetrahedron Lett. 1966, 4765; Gross, H., u. Bilk, L., Liebigs Ann. Chem. 725, 212 (1969)], N-(t-Butyloxycarbonyl)-1H-1,2,4-triazol (Bram, G., Tetrahedron Lett. 1973, 469), t-Butylphenylcarbonat [Ragnarsson, U., Karlsson, S. M., u. Sandberg, B. E., Acta Chim. Scand. 26, 2550 (1972); Org. Synth. 53, 25 (1973)], t-Butyl-S-chinolylcarbonat [Rzesztotarska, B., u. Wiejak, S., Liebigs Ann. Chem. 716, 216 (1968); Rzesztotarska, B.; Wiejak, S., u. Pawelozak, K., Org. Prep. Proc. Int. 5, 71 (1973)], t-Butyl-2,4,5-trichlor-phenylcarbonat (Broadbent, W., Morley, I S., u. Stone, B. E., J. Chem. Soc. (C), 1967, 2632) und Di-t-butyldicarbonat [Tarbell, D. S., Yamamoto, Y., u. Pope, B. M., Proc. Nat. Acad. Sci. USA 69, 730 (1972); Org. Synth. 57, 45 (1977); Moroder, L., Hallet, A., Wünsch, E., Keller, O., u. Wersin, G., Hoppe-Seyler's Z. Physiol, Chem. 357, 1651 (1976)].

Neben diesen häufig angewandten Schutzgruppen werden für spezielle Syntheseprobleme weitere wichtige Schutzgruppen benötigt. Hier haben sich in den letzten Jahren vor allem solche bewährt, die unter milderen Bedingungen abspaltbar sind. Das sind z. B. die 9-Fluorenylmethoxycarbonyl-Gruppen [Carpino, L., J. Am. Chem. Soc. 92, 5748 (1970)], die Methylsulfonylethoxycarbonyl-Gruppe [G. Tesser et al., Intern. J. Peptide Prot. Res. 7, 295 (1975)] oder die 1-Adamantyloxycarbonyl-Schutzgruppe [Wünsch, E., Wackerle, L., u. Moroder, L., Hoppe-Seyler's Z. Physiol. Chem. 357, 1647 (1976)]. Alle diese letztgenannten Schutzgruppen werden gewöhnlich über substituierte Phenole oder N-Hydroxysuccinimidester eingeführt [Bodanszky, M., Klausner, Y. S., u. Ondetti, M. A., in: Peptide Synthesis, 2nd. ed., J. Wiley, N. Y. 1976 p. 32 Gross, H. u. Bilk, L., Liebigs Ann. Chem. 725, 212 (1969); Paquet, A., Can. J. Chem. 57, 2775 (1979) und Can. J. Chem. 60, (8) 976 (1982)].

Die genannten Einführungsreagentien zeichnen sich z. T. dadurch aus, daß bemerkenswert hohe Ausbeuten an urethangeschützten Aminosäuren erreicht werden. Ihr Nachteil besteht jedoch vor allem darin, daß bei der Mehrzahl sowohl die Synthese der Ausgangsstoffe als auch die Herstellung des Einführungsreagens mit hohem präparativen bzw. technischen Aufwand verbunden und ihre Lagerstabilität eingeschränkt ist. Oft ist auch die Aminolysegeschwindigkeit begrenzt.

Bekannt sind auch trägerfixierte Reagentien, die bei Stoffumwandlungsprozessen oder analytischen und präparativen Stofftrennungen verwendet werden (A. Wisemann (Ed.), Handbook of Enzyme Chemistry, New York 1975; D. R. Lowe, An Introduction to Affinity Chromatography, Amsterdam 1979). Das trägerfixierte System besteht aus der polymeren Matrix, dem Träger, an den durch kovalente oder adsorptive Bindung Stoffe mit der gewünschten spezifischen Wirkung (Liganden) gebunden sind. Als Liganden werden natürliche oder synthetische Verbindungen mit bestimmten chemischen oder biologischen Wirkungen verwendet, z. B.

Enzyme als Katalysatoren für Stoffumwandlungen, Proteine und Peptide mit der Wirkung von Lectinen, Inhibitoren, Antigenen oder Antikörpern, Nucleinsäuren, Polysaccharide, Zuckerderivate usw. Die für die Bindung der Liganden verwendeten Polymeren sind natürliche Polysaccharide, wie z. B. Cellulose, Stärke, Dextrane, Agarose und deren Derivate, oder synthetische hydroxylgruppenhaltige Polymere, wie Copolymerisate des 2-Hydroxyethylmethacrylats (Spherone) oder Phenol-Formaldehyd-Kondensationsprodukte (Duolite) sowie aminogruppenhaltige Polymere, wie Polyacrylamide, modifizierte Polysaccharide (z. B. 1H-Sepharose) oder auch Proteine.

Die Art der Knüpfung kovalenter Bindungen zwischen Trägern und Liganden übt einen wesentlichen Einfluß auf die Eigenschaften des entstehenden Produktes aus und war daher immer wieder Gegenstand erneuter Untersuchungen. Am häufigsten erfolgt die Bindung peptidartiger Liganden über deren zugängliche α- und ε-Aminogruppen, obwohl auch die Möglichkeit zur Reaktion der Carboxylgruppen oder der SH-Gruppen besteht.

Voraussetzung für die Bindung der Liganden ist die Aktivierung der chemisch wenig reaktiven Hydroxylgruppen der Träger.

Von vielen möglichen Aktivierungsmethoden hat sich in der Praxis die Umsetzung der Polysaccharide zu Cyanaten oder Imidocarbonaten mittels Bromcyan am meisten durchgesetzt, obwohl damit eine Reihe von Nachteilen verbunden ist (die Reaktion der CNBr-aktivierten Träger mit Aminogruppen führt zu Isoharnstoffderivaten, die den Charakter schwacher Ionenaustauscher erhalten können; hohe Giftigkeit des Bromcyans und seiner Hydrolyseprodukte).

Weiterhin beschrieben wurden die Aktivierung der Hydroxylgruppen mit Benzochinon, Divinylsulfon, Diepoxiden, Trichlortriazin und anderen bifunktionellen Reagentien sowie die Oxidation der Hydroxylgruppen zu Aldehydfunktionen mit Natriumperjodat.

Eine aussichtsreiche Alternative zur Aktivierung mit Bromcyan ist in der Umsetzung von Polysacchariden mit Chlorameisensäureestern zu sehen.

Im Falle der Reaktion de Chlorameisensäureethylesters mit Cellulose [Barker, S. A., Carbohydr. Res. 17, 471 (1971)] ist die Bildung von trans-2,3-cyclischem Carbonat und O-Ethoxycarbonylfunktionen beschrieben, die mit Aminogruppen reagieren. Die Bindung einiger Enzyme und die Darstellung von Immunoadsorbentien ist bekannt [Grey, C. H., Carbohydr. Res. 27, 235 (1973); Kennedy, J. F., J. Immunol. Meth. 50, 57 (1982)].

Die hohe Giftigkeit und die extreme Hydrolyseempfindlichkeit des Chlorameisensäureethylesters sind die Hauptnachteile dieser Aktivierung. Als Vorteile gegenüber der Aktivierung mit Bromcyan ist die hohe Stabilität der durch Umsetzung mit Aminofunktionen entstehenden ungeladenen Urethanbindungen anzusehen. Durch Einsatz reaktionsfähiger und hydrolysebeständigerer Chlorameisensäureester, die in der Peptidchemie eingesetzt werden, wurde die Anwendbarkeit der Methode deutlich verbessert. Die mit Chlorameisensäure- p-nitrophenylester, Chlorameisensäure-N-hydroxysuccinimidester bzw. Chlorameisensäuretrichlorphenylester aktivierten Sepharose-, Spheron- und Celluloseträger sind in getrocknetem Zustand oder in wasserfreiem Dioxan beständig [Drobnik, J., Biotechnol. Bioeng. 24, 487 (1982), Wilchek, M., Miron, T., Biochem. Internat. 4, 629 (1982)].

Entscheidend für die praktische Anwendbarkeit ist die gute Stabilität und Lagerfähigkeit der aktivierten Träger und der für die Gewinnung eingesetzten Chlorameisensäureester. Während die Stabilität der bisher beschriebenen aktivierten Träger befriedigend ist, sind sowohl Chlorameisensäureethylester als auch Chlorameisensäure-N-hydroxysuccinimidester wegen ihrer hohen Hydrolyseempfindlichkeit schlecht handhabbar, insbesondere, wenn in größerem Maßstab gearbeitet werden soll. Nachteile der Chlorameisensäurephenylesterderivate bestehen darin, daß die Abtrennung der bei der Umsetzung mit Aminofunktionen entstehenden, meist giftigen Phenole schwierig ist, andererseits aber sehr gründlich erfolgen muß, da sie die biologische Wirkung der untersuchten Systeme häufig stören. Langwieriges Waschen der hergestellten trägerfixierten Enzyme usw. ist deshalb erforderlich.

Die für die Immobilisierung unterschiedlichster Liganden an polymeren Trägern anwendbaren Methoden sind in Übersichtsarbeiten und Monographien dargestellt (Chemical Analysis, Vol. 59, Elving, P. J., Winefordner, J. D., (Eds.) New York 1981; Hill, E. A., Hirtenstein, M. D., in Advances in Biotechnological Processes 1, p. 31-66, New York, 1983).

Der Erfindung liegt die Aufgabe zugrunde, ein neues, stabiles Ausgangsprodukt und Verfahren zu seiner Herstellung sowie zu seiner Anwendung zur Verfügung zu stellen, das sich zur vorteilhaften Herstellung unsymmetrischer Carbonate eignet, die ihrerseits zur Einführung von Urethan-Schutzgruppen in Aminosäuren Verwendung finden können, und das ferner eine einfache und wirtschaftliche Herstellung aktivierter Träger zur Bindung nucleophiler Komponenten sowie stabiler Träger-Ligand-Komplexe ermöglicht.

Die Aufgabe wird gemäß des Ansprüches 1, 2 und 4 gelöst. Anspruch 3 betrifft eine vorteilhafte Weiterbildung.

Der erfindungsgemäße neue Chlorameisensäureester ist das N-Chlorcarbonyloxy-5-norbornen-2,3-dicarboximid (Cl-CO-ONB) der Formel I. Durch Umsetzung von Phosgen mit N-Hydroxy-5-norbornen-2,3-dicarboximid (HONB) der Formel II oder mit dem Natriumsalz der N-Hydroxy-Verbindung (NaONB) der Formel III wird die gewünschte Verbindung Cl-CO-ONB (I) nach folgendem Reaktionsschema erhalten:

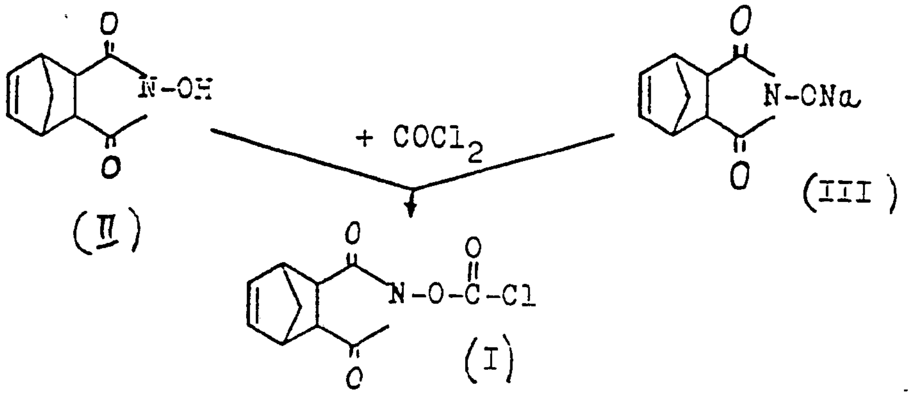


Die Verwendbarheit der Verbindung I geht aus den folgenden Reaktionsschemata 1 und 2 hervor:

**Reaktionsschema 1**

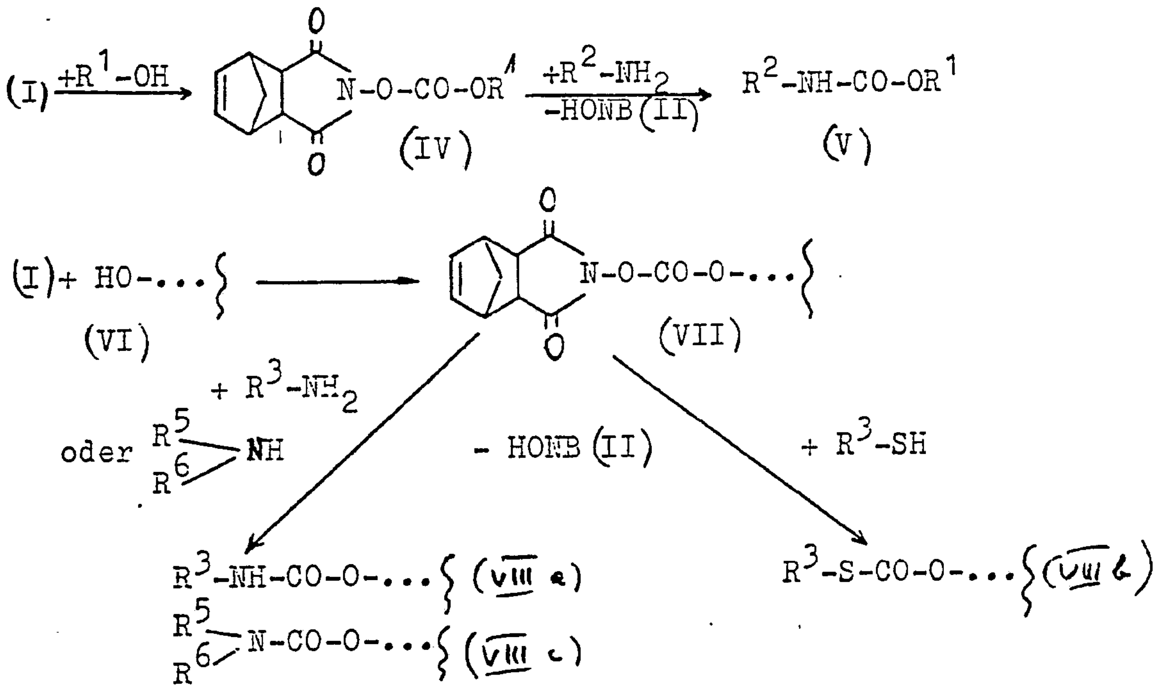

**Reaktionsschema 2**

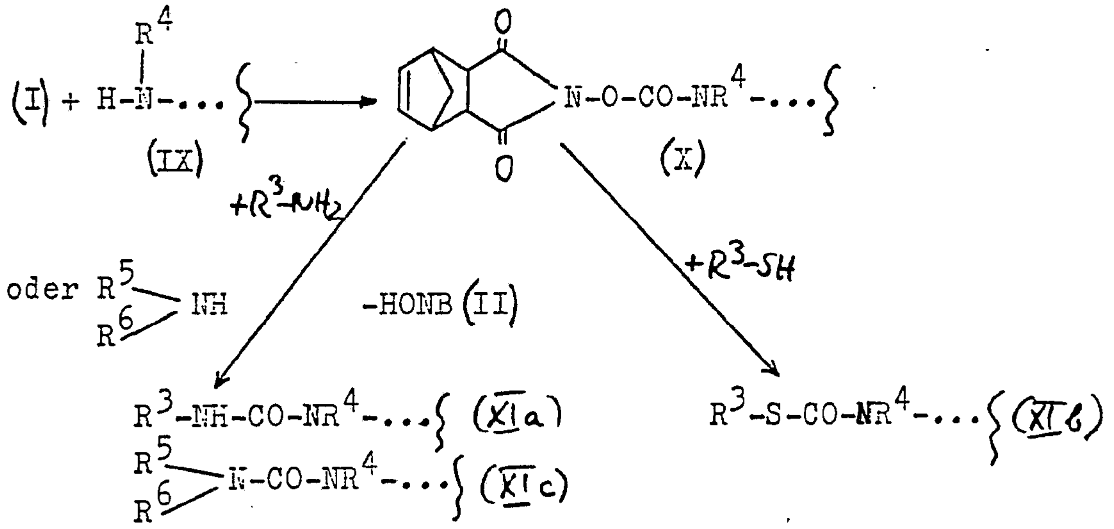


Wie aus Reaktionsschema 1 hervogeht, lassen sich unsymmetrische Carbonate der Formel IV, in denen $R^1$ für t -Butyl-1-adamantyl, 2-Diphenylpropyl, 9-Methylfluorenyl, Methylsulfonylethyl, Benzyl oder andere in der Peptidchemie übliche Reste steht, aus dem erfindungsgemäß hergestellten Cl-CO-ONB (I) mit den entsprechenden Alkoholen $R^1$-OH gewinnen (vgl. auch das nachstehende Formelschema).

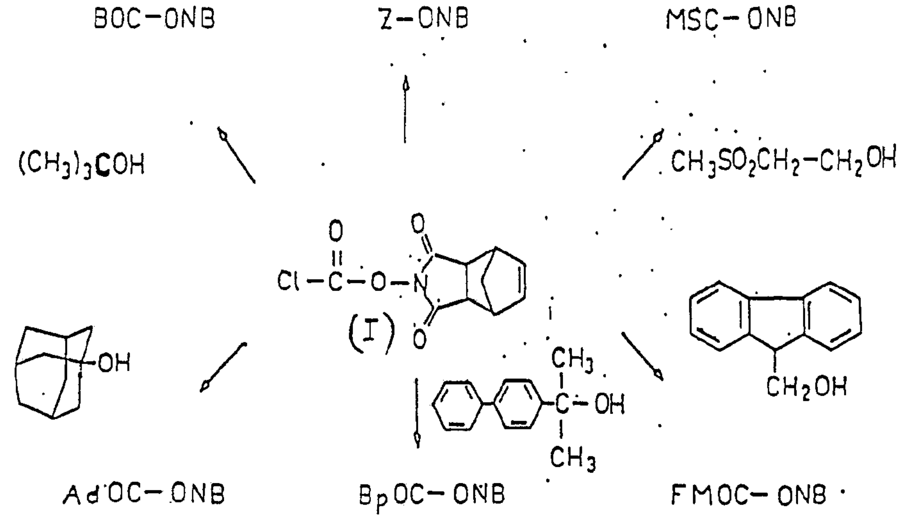


Beispiele hierzu sind im experimentellen Teil angegeben (Tabelle 1).

Aus den unsymmetrischen Carbonaten der Formel IV erhält man durch Umsetzung mit Aminosäuren bzw. Peptiden, die eine ungeschützte Aminogruppe enthalten, urethangeschützte Aminosäuren bzw. Peptide der Formel V, in denen $R^2$ für Aminosäure- bzw. Peptidreste steht. Beispiele hierzu sind im experimentellen Teil angeführt (Tabelle 2).

Die Verwendung des Chlorameisensäureesters I zur Herstellung aktivierter Träger der Formeln VII und X erfolgt dadurch, daß die Verbindung I mit hydroxylgruppenhaltigen Polymeren der Formel VI (Reaktionsschema 1) oder aminogruppenhaltigen unlösliche Polymere der Formel IX, in der $R^4$ für H, Alkyl, substituiertes Alkyl, Aryl oder substituiertes Aryl steht (Reaktonsschema 2), umgesetzt wird.

Die entstehenden aktivierten Träger VII sind dadurch gekennzeichnet, daß die zugänglichen Hydroxylgruppen in einem weit variierbaren Maße durch die -O-CO-ONB-Gruppierung bzw. die Aminogruppen der Träger IX durch die Gruppierung -NH-CO-ONB ersetzt sind.

Die Umsetzung der Träger VI und IX mit der Verbindung I erfolgt in wasserfreien organischen Lösungsmitteln, wie 1,4-Dioxan, Aceton, Pyridin, Ether usw. Durch Waschen des so entstandenen Produktes mit Dioxan, Ether oder Aceton werden nichtumgesetztes N-Chlorcarbonyloxy-5-norbornen-2,3-dicarboximid I und freigesetzter Chlorwasserstoff entfernt.

Im allgemeinen verläuft die Reaktion in gleicher Weise, wenn die Umsetzung in Gegenwart von tertiären

Aminen zur Bindung des entstehenden Chlorwasserstoffs durchgeführt wird. Der Vorteil des Basenzusatzes ist darin zu sehen, daß die vom aktivierten Träger durch Filtration abgetrennte Lösung des Chlorameisensäureesters I erneut für die Umsetzung mit dem Träger verwendet werden kann, ohne daß die Aktivierungsrate herabgesetzt ist. Durch die wiederholte Verwendung der Lösung des Chlorameisensäureesters I wird die Wirtschaftlichkeit des Aktivierungsverfahrens erheblich verbessert. Die aktivierten Träger können nach Entfernen des Lösungsmittels in getrocknetem Zustand oder in einem wasserfreien Lösungsmittel, wie Dioxan, Ether, Chloroform etc., bei niedriger Temperatur über Monate ohne Aktivitätsverluste aufbewahrt werden.

Auch in wäßrigen Lösungen sind die durch Umsetzung mit der Verbindung I erhaltenen Träger weitgehend stabil.

Der Vorteil der erfindungsgemäß erhältlichen aktivierten Träger besteht in einer höheren Stabilität im alkalischen Milieu. Durch die geringe Hydrolysegeschwindigkeit der aktivierten Träger wird es möglich, die Kopplung mit nucleophilen Reagentien bei vergleichsweise hohen pH-Werten vorzunehmen.

Als hydroxylgruppenhaltige Träger der Formel VI können natürliche Polysaccharide, wie Cellulose in den unterschiedlichsten Formen, Stärke, Stärkehydrolyseprodukte (SHP), vernetzte Dextrane und Agarose, sowie synthetische Polymere, wie Polyvinylalkohol (PVA)/Duolite, Spherone, hydrophile Vinylpolymere (Fractogel TSK) usw., aber auch anorganische Träger, wie z. B. poröses Glas, verwendet werden.

Weiterhin lassen sich auch aminogruppenhaltige unlösliche Polymere der Formel IX, wie z. B. Aminoalkylcellulose, Aminoalkylpolystyrol, Melamin- und Harnstoff-Formaldehyd- Harze sowie Polyacrylamid als Träger einsetzen.

Zusätzliche Vorteile bestehen in der leichten Zugänglichkeit des verwendeten Chlorameisensäureesters I und der guten Lagerfähigkeit der aktivierten Träger, was auch Umsetzungen in größerem Maßstab gestattet. Hervorzuheben ist, daß die im Überschuß eingesetzte Lösung des Chlorameisensäureesters I mehrfach für Umsetzungen ohne erneute Reinigung eingesetzt werden kann. Ausbeuteverluste an aktiviertem Träger sind nicht erkennbar.

Die so erhaltenen Träger zeichnen sich durch eine hohe Reaktionsgeschwindigkeit beim Umsatz mit Aminogruppen und SH-Gruppenenthaltenden Liganden aus (vgl. die obigen Reaktionsschemata 1 und 2). Die bei der Umsetzung mit Nucleophilen entstehenden Träger-Ligand-Komplexe der Formeln VIII a-c und XI a-c, in denen $R^3$ für H, Alkyl, substituiertes Alkyl, Aryl, substituiertes Aryl, einen Aminosäure-, Peptid- oder Proteinrest und $R^5$ sowie $R^6$ für H, Alkyl, substituiertes Alkyl, Aryl oder substituiertes Aryl stehen, weisen eine hohe Stabilität auf. Ein Vorteil bei der Immobilisierung der Liganden besteht darin, daß das bei der Kopplung entstehende N-Hydroxy-5-norbornen-2,3-dicarboximid (HONB) II infolge seiner sehr guten Wasserlöslichkeit leicht entfernt werden kann. Ein weiterer Vorteil - gegenüber den Hydroxysuccinimidestern - ist, daß bei der Hydrolyse von Chlorameisensäureester aktivierten Polymeren keine geladenen Gruppen entstehen, sondern die ursprünglich vorhandenen Hydroxylgruppen des Trägers zurückgebildet werden. Gegenüber bisher verwendeten Chlorameisensäureestern ist die Hydrolyse der mit der Verbindung I aktivierten Polymeren wenig ausgeprägt. Auf diese Weise ist es gelungen, die Nachteile bisher bekannter Methoden zur Herstellung von Träger-Ligand-Komplexen weitgehend zu beseitigen.

Bei Verwendung von geeigneten Resten $R^3$, wie z. B. H, $CH_2$-$CH_2$-$N(R^7)_2$ mit $R^7$ = H, $CH_3$, $C_2H_2$, $(CH_2$-$CH_2$-$NH)_nH$ mit n = 1 bis 500, C(NH)-$NH_2$, $C_6H_4$-$NH_2$ oder $CH_2$-COOH ist Cl-CO-ONB I auch zur Herstellung von Ionenaustauschern geeignet.

Die unsymmetrischen Carbonate IV lassen sich auch ohne Isolierung des Cl-CO-ONB I nach der Umsetzung von HONB II mit Phosgen in einem Eintopfverfahren herstellen (Reaktionsschema 1).

Die Umsetzung hydroxylgruppenhaltiger Träger VI, wie Cellulosen, z. B. in Form von Papier, Fasern, Pulvern oder Perlen, ferner von Stärkehydrolyseprodukten (SHP), vernetzten Dextranen, Agarose und Polyvinylalkoholen, Duoliten, Fractogelen oder Spheronen mit I erfolgt in wasserfreien organischen Lösungsmitteln, ggfs. in Gegenwart von Basen. Durch Waschen des so entstandenen aktivierten Trägers lassen sich nichtumgesetzte Verbindung I und freigesetzter Chlorwasserstoff entfernen, wobei die Lösung des Chlorameisensäureesters mehrfach für Umsetzungen ohne erneute Reinigung eingesetzt werden kann. Der aktivierte Träger wird in wasserfreien Lösungsmitteln oder in lyophilisierter Form aufbewahrt. In analoger Weise erfolgt die Umsetzung von aminogruppenhaltigen polymeren Trägern IX mit Verbindung I (Reaktionsschema 2). Die Träger-Ligand-Komplexe der Formeln VIII a-c und XI a-c werden durch kovalente Bindung nucleophiler Liganden an durch Cl-CO-ONB I aktivierte hydroxylgruppenhaltige bzw. aminogruppenhaltige Verbindungen gebildet. Beispiele für geeignete hydroxylgruppenhaltige Verbindungen sind etwa natürliche Polysaccharide, wie Agarosen, Dextrane, Cellulosen, z. B. Papier, Zellstoff, Cellulosepulver und Celluloseperlen, Stärke und Stärkehydrolyseprodukte (SHP), chemisch modifizierte Polysaccharide, und synthetische Polymere, wie Polyvinylalkohole, Duolite, Spherone usw., und Oligomere. Beispiele für geeignete aminogruppenhaltige unlösliche Polymere sind etwa Aminoalkylcellulosén, aminoalkylierte Polystyrole, Harnstoff-Formaldehyd-Harze und Melamin-Formaldehyd-Harze und Polyacrylamide. Als nucleophile Liganden finden Proteine, Enzyme, Nucleinsäuren, Aminosäuren, Nucleotide, Antigene und Antikörper, Hormone, Rezeptoren, SH-Gruppenenthaltende Verbindungen, $NH_3$ oder Amine Verwendung. Das bei der Umsetzung von Träger und Ligand entstehende HONB II wird durch Waschen mit wäßrigen Lösungsmitteln entfernt.

Die Erfindung wird im folgenden anhand von Ausführungsbeispielen näher erläutert.

Es werden folgende Abkürzungen verwendet:

| | |
|---|---|
| Adoc-ONB | 1-Adamantyl-oxycarbonyl-ONB |
| AS | Aminosäure |
| Boc-ONB | t-Butyloxycarbonyl-ONB |
| Bpoc-ONB | 2-Diphenylpropoxycarbonyl-ONB |
| Cl-CO-ONB | N-Chlorcarbonyloxy-5-norbornen-2,3-dicarboximid |
| Fmoc-ONB | 9-Fluorenylmethoxycarbonyl-ONB |
| HONB | N-Hydroxy-5-norbornen-2,3-dicarboximid |
| Msc-ONB | Methylsulfonylethoxycarbonyl-ONB |
| NaONB | Natriumsalz des HONB |
| - ONB | -oxy-5-norbornen-2,3-dicarboximid |
| SHP | Stärkehydrolyseprodukte |
| THF | Tetrahydrofuran |
| TNBS | Trinitrobenzolsulfonsäure |
| Z-ONB | Benzyloxycarbonyl-ONB |

**Beispiel 1:**

Herstellung von N-Chlorcarbonyloxy-5-norbornen-2,3-dicarboximid

Beispiel 1.1.

Zu einer Lösung von Phosgen (9,9 g; 0,1 mol) wird tropfenweise eine Lösung aus 17,9 g HONB, 12 g N,N-Dimethylanilin in THF/Benzol (etwa 200 ml) bei 0 bis 5°C zugesetzt. Anschließend wird 1 h bei dieser Temperatur gerührt und über Nacht stehengelassen, worauf das Hydrochlorid abgetrennt und das Lösungsmittel im Vakuum verdampft wird. Der feste Rückstand wird in 50 ml THF aufgenommen, auf -20°C abgekühlt, filtriert und erneut im Vakuum eingeengt.

F. 98 - 100°C (Zers.)

Ausbeute: 95 %.

Massenspektrometrischer Molekülpeak bei 241.

IR (1740 $cm^{-1}$ Cl-CO; 1800 $cm^{-1}$, 1820 $cm^{-1}$ Imidocarbonyl).

Beispiel 1.2.

9,9 g (55 mmol) HONB werden mit 55 ml methanolischer 1 N NaOH versetzt. Nach kurzer Zeit beginnt das Na-Salz auszufallen. Die Fällung des Na-Salzes wird durch Zugabe von 100 ml Diethylether vervollständigt. Das Salz wird abgesaugt und über $P_4O_{10}$ getrocknet. Ausbeute 9,85 g.

9,85 g des getrockneten Na-Salzes werden portionsweise unter Rühren zu einer Lösung von 55 mmol Phosgen in 200 ml Toluol bei Raumtemperatur zugesetzt. Es wird über Nachtweiter gerührt und anschließend vom ausgefallenen NaCl abfiltriert. Das Filtrat wird eingeengt und der verbleibende Feststoff in wenig THF (50 ml) aufgenommen. Das Lösungsmittel wird erneut im Vakuum abgetrennt. Der verbleibende Feststoff ist mit dem unter Beispiel 1.1. gewonnenen Produkt identisch.

**Beispiel 2:**

Herstellung unsymmetrischer Carbonate IV

Beispiel 2.1.

0,1 mol N-Chlorcarbonyloxy-5-norbornen-2,3-dicarboximid (Cl-CO-ONB) I wird in 150 ml eines inerten Lösungsmittels (z. B. Toluol, Benzol, THF, halogenierte Kohlenwasserstoffe) gelöst und bei 10 bis 15°C mit einer Lösung von 0,1 mol eines Alkohols (Tabelle 1) und 0,1 mol eines tertiären Amins in 40 ml eines inerten Lösungsmittels (wie oben angegeben) versetzt. Anschließend rührt man 1 h bei Raumtemperatur und 3 h bei 35°C. Nach Stehenlassen über Nacht wird der aus Aminhydrochlorid bestehende Niederschlag abgetrennt und das Filtrat eingeengt. Zur Abtrennung von Resten des Amins ist es auch möglich, die Lösung vor dem Einengen mit eiskalter 5 %-iger $NaHCO_3$-Lösung zu waschen (Tabelle 1).

Beispiel 2.2.

Zu einer Lösung von Phosgen (9,9 g; 0,1 mol) wird tropfenweise eine Lösung aus 17,9 g HONB und 12 g N,N-Dimethylanilin in THF/Benzol (etwa 200 ml) bei 0 bis 5°C zugegeben. Anschließend wird noch 1 h bei dieser Temperatur gerührt und über Nacht stehengelassen. Dann wird ohne Isolierung des Cl-CO-ONB wie unter Beispiel 2.1. angegeben weiter verfahren.

**Tabelle 1:** Unsymmetrische Carbonate

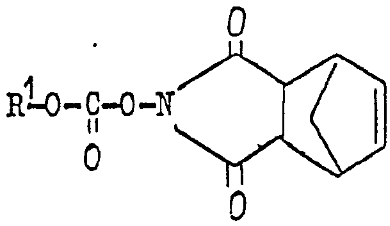

(IV)

| $R^1$ | F. (°C) | Molmasse | Summenformel | Elementaranalyse ber.: C (%) / gef.: C (%) | H (%) / H (%) | N (%) / N (%) | Massenspektrometrischer Molkülpeak ($M^+$) |
|---|---|---|---|---|---|---|---|
| Adamantyl | 220 | 357,1 | $C_{20}H_{23}NO_5$ | 67,21 | 6,49 | 3,92 | |
| | | | | 67,43 | 6,52 | 3,87 | 357,1 |
| t-Butyl | 124 | 279,3 | $C_{14}H_{17}NO_5$ | 60,20 | 6,10 | 5,01 | |
| | | | | 60,50 | 6,07 | 5,17 | 279,3 |
| 9-Methylfluorenyl | 129 | 401,1 | $C_{24}H_{19}NO_5$ | 71,81 | 4,77 | 3,49 | 401,4 |
| | | | | 71,7 | 4,5 | 3,57 | |
| $CH_3$-S-$CH_2$-$CH_2$ | 145 | 313,26 | $C_{13}H_{15}NO_6S$ | 49,84 | 4,83 | 4,47 | |
| | | | | 49,87 | 4,73 | 4,31 | 313 |
| 2-Diphenylpropyl | 121 | 417,4 | $C_{25}H_{23}NO_5$ | 71,99 | 5,55 | 3,36 | |
| | | | | 71,80 | 5,42 | 3,21 | 417,4 |

**Beispiel 3:**

Herstellung urethangeschützter Aminosäuren und Peptide V

Die jeweilige Aminosäure wird in einem Gemisch aus Wasser/ Aceton, Wasser/Dioxan oder Wasser/t-Butanol gelöst (evtl. suspendiert) und mit der zwei- bis vierfachen molaren Menge an Triethylamin versetzt. Im Anschluß daran wird das unsymmetrische Carbonat IV in einem Überschuß von 10 % zugegeben. Man rührt bei Raumtemperatur mehrere Stunden (abhängig von der Aminosäure, bei Einführung der Bpoc-Schutzgruppen beträgt die Reaktionstemperatur 45°C). Der organische Lösungsmittelanteil wird im Vakuum entfernt und der verbleibende Rückstand mit 10 %-iger $KHSO_4$-Lösung angesäuert.

Man verteilt zwischen Methylenchlorid und $KHSO_4$-Lösung und trennt die organische Phase ab. Die organische Phase wird zur Entfernung des restlichen HONB gründlich mit Wasser gewaschen und über MgSO getrocknet. Dann wird eingeengt und n-Hexan verrieben (Tabelle 2).

**Beispiel 4:**

Herstellung aktivierter Träger VII

Als Ergebnis einer Aktivierung der Träger wurde die kovalente Bindung von Glycin nach dem Aktivierungsschritt bestimmt. Es wurde der Verbrauch an zugesetztem Glycin nach der TNBS-Methode (Trinitrobenzolsulfonsäure, Reagens für $NH_2$-Gruppen) ermittelt.

Beispiel 4.1:

Zu 1 ml in wasserfreiem Dioxan suspendierter Perlcellulose wurden 14 ml Lösung des Esters Cl-CO-ONB I (Konz: 38 mg/ml) zugegeben. Die Suspension wurde 3 h bei 60°C geschüttelt. Der abfiltrierte Träger wurde mit wasserfreiem Dioxan gewaschen und mit 5 ml Glycinlösung (Konz: 4 mg/ml ≙ 53,4 µmol/ ml) in 0,1 M Na-Tetraboratpuffer pH 8 versetzt. Nach zweistündigem Schütteln bei Raumtemperatur wurde der Träger abgesaugt und im Überstand der Glycinverbrauch ermittelt.

Ergebnis: 49,3 µmol Glycin/ml Träger.

**Tabelle 2:** Urethangeschützte Aminosäuren bzw. Peptide

| | Derivat AS/Peptide | F. (°C) | Ausbeute (%) |
|---|---|---|---|
| 1. Boc-ONB | Boc-Ala-OH | 82 | 87 |
| | Boc-Gly-OH | 85-87 | 85 |
| | Boc-Leu-OH | 79-83 | 91 |
| | Boc-Pro-OH | 132-33 | 88 |
| | Boc-Trp-OH | 136-38 | 86 |
| | Boc-β-Ala-OH | 79-81 | 84 |
| | Boc-Phe-OH | 82-84 | 91 |
| 2. Fmoc-ONB | | | |
| | Fmoc-Ala-OH | 144 | 95 |
| | Fmoc-Gly-OH | 172-74 | 87 |
| | Fmoc-Leu-OH | 153-55 | 91 |
| | Fmoc-Phe-OH | 180-82 | 95 |
| | Fmoc-Val-OH | 142-44 | 85 |
| | Fmoc-Ser-OH | 86-88 | 87 |
| | Fmoc-Trp-Met-OH | 138 | 79 |
| | Fmoc-Met-Gly-OH | 110-112 | 77 |
| 3. Adoc-ONB | | | |
| | Adoc-Phe-OH | 60-63 | 82 |
| | Adoc-Ala-OH | 140-41 | 84 |
| | Adoc-Gly-OH | 141-42 | 87 |
| 4. Bpoc-ONB | | | |
| | Bpoc-Pro-OH | 122-124 | 81 |
| | Ppoc-Phe-OH | 85-87 | 85 |
| 5. Msc-ONB | | | |
| | Msc-Phe-OH | 112-113 | 89 |
| | Msc-Ile-OH | 92-95 | 81 |

Beispiel 4.2.:

Sepharose 4B CL wurde in der gleichen Weise wie in Beispiel 4.1. aktiviert und auch nach gleichem Verfahren mit Glycinlösung behandelt.

Ergebnis: 33,3 µmol Glycin/ml Träger.

Beispiel 4.3.:

Spheron P 1000 wurde in der gleichen Weise wie in Beispiel 4.1. aktiviert und auch nach gleichem Verfahren mit Glycinlösung behandelt.

Ergebnis: 49,3 µmol Glycin/ml Träger.

Beispiel 4.4.:

Perlcellulose wurde bei 80°C aktiviert unter Beibehaltung aller anderen im Beispiel 4.1. aufgeführten Bedingungen, einschließlich der Kopplung mit Glycin.

Ergebnis: 69,3 µmol Glycin/ml Träger.

Beispiel 4.5.:

Perlcellulose wurde wie in Beispiel 4.1. aktiviert, wobei der Lösung des Esters I 10 mg Triethylamin als Base pro ml Träger zugesetzt wurden. Die Kopplung mit Glycin wurde wie in Beispiel 4.1. durchgeführt.

Ergebnis: 41,3 µmol Glycin/ml Träger.

Beispiel 4.6.:

Die aus Beispiel 4.5. zurückgewonnene Esterlösung wurde erneut zur Aktivierung verwendet (Bedingungen wie in Beispiel 4.5. unter nochmaligem Basenzusatz zur Esterlösung von 10 mg/ml Träger).

Ergebnis: 56,0 µmol Glycin/ml Träger.

Beispiel 4.7.:

1 ml Perlcellulose, suspendiert in wasserfreiem Dioxan, wurde 6 h bei 80°C mit 3,5 ml Lösung des Esters I (Konz: 200 mg/ml) geschüttelt. Nach Filtrieren und Waschen mit wasserfreiem Dioxan wurden dem Träger 5 ml Glycinlösung (Konz: 4 mg/ml ≙ 53,4 µmol/ml) in 0,1 M Na-Tetraboratpuffer pH 8 zugesetzt. Die Suspension wurde 2 h bei Raumtemperatur und anschließend 22 h bei 4°C geschüttelt. Nach Absaugen des Trägers wurde der Glycingehalt im Überstand ermittelt und die Differenz zur eingesetzten Menge berechnet.

Ergebnis: 116 µmol Glycin/ml Träger.

Beispiel 4.8.:

1 ml Perlcellulose, suspendiert in wasserfreiem Dioxan, wurde 3 h bei 70°C mit 1,7 ml Esterlösung (Konz.: 300

mg/ml) geschüttelt. Nach Filtration und Waschen des Trägers mit wasserfreier Dioxanlösung wurden 5 ml Glycinlösung (Konz.: 4 mg/ml = 53,4 µmol/ml) in 0,1 M Phosphatpuffer pH 8 zugegeben und bei Raumtemperatur 2 h geschüttelt. Der Glycinverbrauch wurde bestimmt.

Ergebnis: 116,7 µmol Glycin/ml Träger.

Beispiel 4.9.:

1 ml Perlcellulose wurde wie in Beispiel 4.8. behandelt. Lediglich das Glycin wurde in 0,5 M $NaHCO_3$ zugegeben und die Kopplung 24 h bei 4° C durchgeführt.

Ergebnis: 150,7 µmol Glycin/ml Träger.

Beispiel 4.10.:

100 mg Zellstoff (trocken) wurden mit 30 ml Lösung des Esters I (Konz.: 37 mg/ml) 3 h bei 60° C akiviert. Die Kopplung mit Glycin erfolgte 2 h bei Raumtemperatur mit 10 ml Lösung (Konz: 4 mg Glycin/ml 0,1 M Na-Boratpuffer pH 8) unter leichtem Schütteln.

Ergebnis: 4,78 mg Glycin/100 mg Zellstoff ≙
63,7 µmol Glycin/100 mg Zellstoff.

**Beispiel 5:**

Herstellung von Träger-Ligand-Komplexen VIII

Als Beispiel für Ligandenbindung wurde die Kopplung von Ovomucoid und Concanavalin A (ConA) durchgeführt, wobei die gebundene Proteinmenge nach 18 h Behandlung der Träger mit 1 N NaoH nach Lowry et al. [J. Biol. Chem. 193, 265 (1951)] bestimmt wurde.

Beispiel 5.1.:

In 1 ml wasserfreiem Dioxan suspendierter Perlcellulose wurden 14 ml Lösung des Esters Cl-CO-ONB I (Konz.: 38 mg/ml) zugegeben. Die Suspension wurde 3 h bei 60° C geschüttelt. Der abfiltrierte Träger wurde mit wasserfreiem Dioxan gewaschen und mit 1,2 ml Ovomucoidlösung in 0,1 M Na-Tetraboratpuffer pH 8 (Ovomucoideinsatz: 10 mg/ml Träger) versetzt. Die Kopplung wurde 2 h bei Raumtemperatur unter Schütteln durchgeführt.

Ergebnis: 1,24 mg Ovomucoid/ml Träger.

Beispiel 5.2.:

Der gleiche Versuch wie in Beispiel 5.1. wurde mit Sepharose 4 B Cl durchgeführt.

Ergebnis: 2,48 mg Ovomucoid/ml Träger.

Beispiel 5.3.:

Der gleiche Versuch wie in Beispiel 5.1. wurde mit Spheron P 1000 durchgeführt.

Ergebnis: 1,45 mg/Ovomucoid/ml Träger.

Beispiel 5.4.:

Der Versuch wurde wie in Beispiel 5.1. durchgeführt, wobei jedoch ein Ovomucoideinsatz von 100 mg/ml Träger angewandt wurde.

Ergebnis: 18,6 mg Ovomucoid/ml Träger.

Beispiel 5.5.:

Der Versuch wurde wie in Beispiel 5.1. durchgeführt, wobei die Ovomucoidkopplung jedoch 2 h bei Raumtemperatur und anschließend noch 24 h bei 4° C durchgeführt wurde.

Ergebnis: 7 mg Ovomucoid/ml Träger.

Beispiel 5.6.:

Die Aktivierungsbedingungen wurden wie folgt geändert: 6 h bei 80° C mit 3,5 ml Lösung des Esters I (Konz.: 200 mg/ml pro ml Perlcellulose). Die Kopplung erfolgte durch Zugabe von 1,2 ml Ovomucoidlösung (Konz.: 41,66 mg Ovomucoid/ml) und 2 h Schütteln bei Raumtemperatur und 22 h bei 4° C.

Ergebnis: 13,8 mg Ovomucoid/ml Träger.

Beispiel 5.7.:

Die Aktivierung von 1 ml Perlcellulose erfolgte wie in Beispiel 5.1. Zur Kopplung wurden 1,3 ml Concanavalin A-Lösung (10 mg Concanavalin A) in 0,1 M Na-Tetraboratpuffer pH 8 zugegeben. Geschüttelt wurde 5 h bei Raumtemperatur.

Ergebnis: 6,7 mg ConA/ml Träger.

**Patentansprüche**

1. N-Chlorcarbonyloxy-5-norbornen-2,3-dicarboximid der Formel I

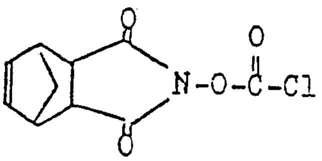

(I).

2. Verfahren zur Herstellung von N-Chlorcarbonyloxy-5-norbornen-2,3-dicarboximid der Formel 1 nach Anspruch 1,
gekennzeichnet durch
Umsetzung von N-Hydroxy-5-norbornen-2,3-dicarboximid der Formel II oder dessen Natriumsalz der Formel III mit Phosgen nach folgendem Reaktionsschema:

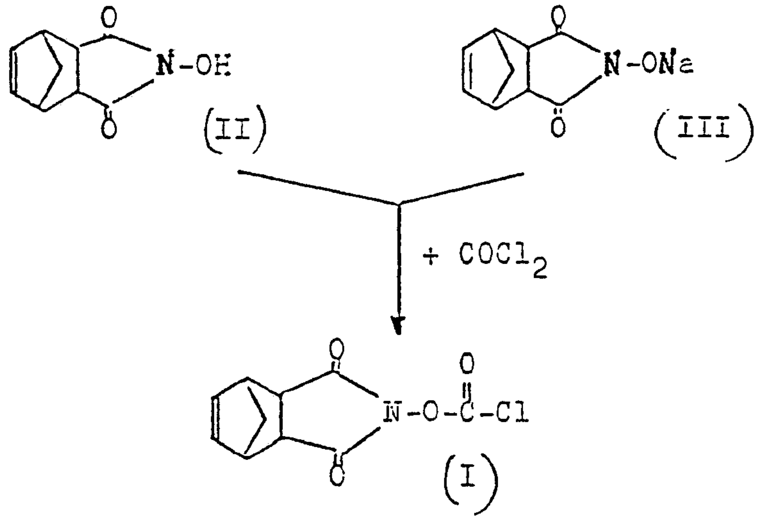


3. Verfahren nach Anspruch 2, dadurch gekennzeichnet, daß die Umsetzung des N-Hydroxy-5-norbornen-2,3-dicarboximids mit Phosgen in Gegenwart tertiärer Amine vorgenommen wird.

4. Verwendung des N-Chlorcarbonyloxy-5-norbornen-2,3-dicarboximids der Formel I nach Anspruch 1 zur Herstellung von
- unsymmetrischen Carbonaten der Formel IV

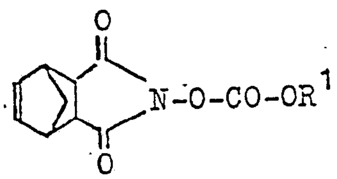

(IV),

worin $R^1$ t-Butyl 1-Adamantyl, 2-Diphenylpropyl, 9-Methylfluorenyl, Methylsulfonylethyl, Benzyl oder andere in der Peptidchemie übliche Reste bedeutet,
- urethangeschützten Aminosäuren bzw. Peptiden der Formel V
$R^2$-NH-CO-O$R^1$ (V),
worin $R^1$ dasselbe wie oben
und
$R^2$ einen Aminosäure- oder Peptidrest bedeuten,
- aktivierten Trägern der Formeln VII

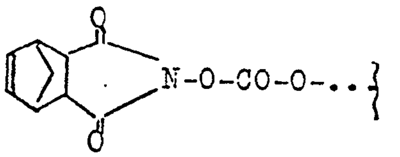 (VII)

und X

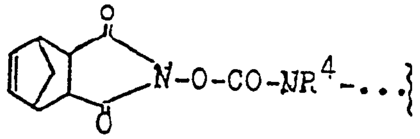 (X),

worin $R^4$ H, Alkyl, substituiertes Alkyl, Aryl oder substituiertes Aryl bedeutet,
sowie
Träger-Ligand-Komplexen der Formeln VIIIa, VIIIb, VIIIc, XIa, XIb und XIc:
$R^3$-NH-CO-O-...(VIIIa),
$R^3$-S-CO-O-...(VIIIb),
worin $R^3$ H, Alkyl, substituiertes Alkyl, Aryl oder substituiertes Aryl oder einen Aminosäure-, Peptid- oder Proteinrest bedeutet,

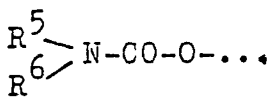 (VIIIc),

worin $R^5$ und $R^6$ H, Alkyl, substituiertes Alkyl, Aryl oder substituiertes Aryl bedeuten,
$R^3$-NH-CO-$NR^4$-...(XIa),
$R^3$-S-CO-$NR^4$-...(XIb)
und

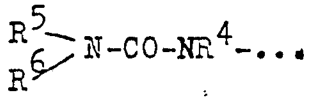 (XIc)

worin $R^3$, $R^4$, $R^5$ und $R^6$ die gleiche Bedeutung wie oben haben.

## Claims

1. N-Chlorocarbonyloxy-5-norbornene-2,3-dicarboximide of the formula I

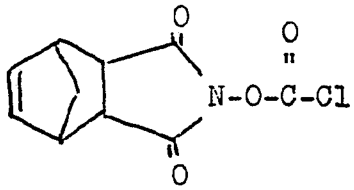 (I)

2. Process for the preparation of N-chlorocarbonyloxy-5-norbornene-2,3-dicarboxmide of formula I according to claim 1, characterised by the reaction of N-hydroxy-5-norbornen-2,3-dicarboximide of the formula II or of its sodium salt of the formula III with phosgene according to the following reaction scheme:

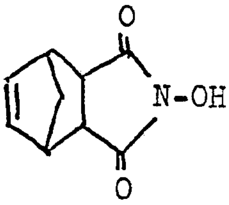

(II)

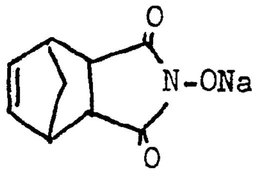

(III)

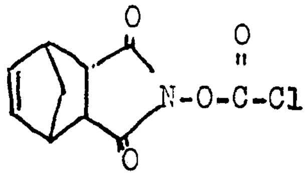

(I)

3. Process according to claim 2, characterised in that the reaction of the N-hydroxy-5-norbornene-2,3-dicarboximide with phosgene is carried out in the presence of tertiary amines.

4. Use of the N-chlorocarbonyloxy-5-norbornene-2,3-dicarboxyimide of the formula I according to claim 1 for the preparation of

- asymmetric carbonates of the formula IV

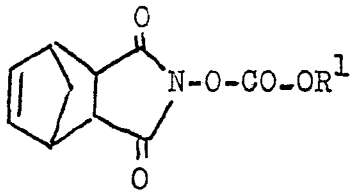

(IV),

wherein $R^1$ signifies t-butyl, 1-adamantyl, 2-diphenylpropyl, 9-methylfluorenyl, methylsulphonylethyl, benzyl or other radicals usual in peptide chemistry,

- urethane-protected amino acids or peptides of the formula V

$R^2$-NH-CO-O$R^1$ (V)

wherein $R^1$ signifies the same as above and $R^2$ an amino acid or peptide residue,

- activated carriers of the formulae VII

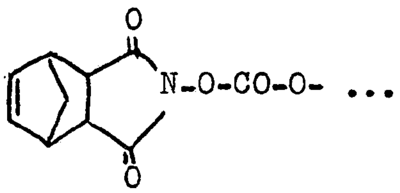

(VII)

and X

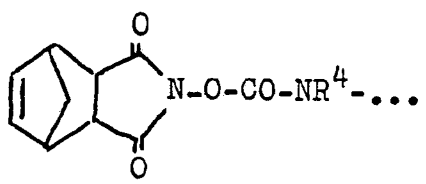

(X)

wherein $R^4$ signifies H, alkyl, substituted alkyl, aryl or substituted aryl, as well as

- carrier-ligand complexes of the formulae VIIIa, VIIIb, VIIIc, XIa, XIb and XIc:

$R^3$-NH-CO-O-... (VIIIa),

$R^3$-S-CO-O-.. (VIIIb),
wherein $R^3$ signifies H, alkyl, substituted alkyl, aryl or substituted aryl or an amino acid, peptide or protein residue,

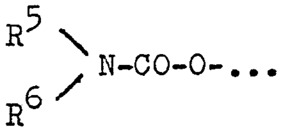
(VIIIc),

wherein $R^5$ and $R^6$ signify H, alkyl, substituted alkyl, aryl or substituted aryl,
$R^3$-NH-CO-N$R^4$-.. (XIa)
$R^3$-S-CO-N$R^4$-.. (XIb)
and

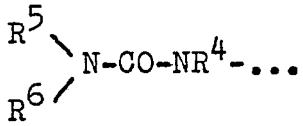
(XIc)

wherein $R^3$, $R^4$, $R^5$ and $R^6$ have the same meaning as as above.

## Revendications

1. N-chlorocarbonyloxy-5-norbornène-2,3-dicarboximide de formule I

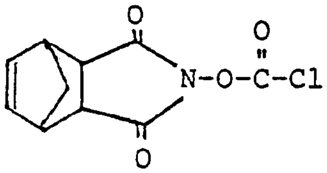
(I)

2. Procédé de préparation de la N-chlorocarbonyloxy-5-norbornène-2,3-dicarboximide de formule I selon la revendication 1, caractérisé par la réaction de la N-hydroxy-5-norbornène-2,3-dicarboximide de formule II ou de son sel sodique de formule III avec du phosgène selon le schéma réactionnel suivant:

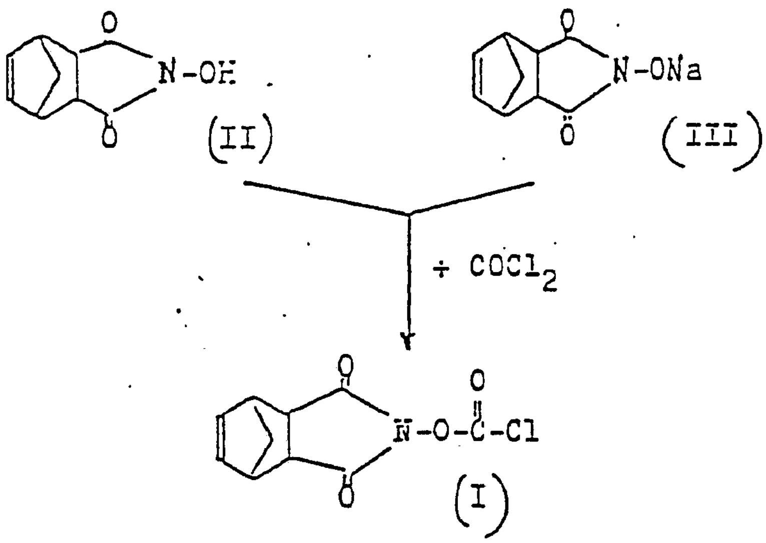


3. Procédé selon la revendication 2, caractérisé en ce qu'on entreprend la réaction de la N-hydroxy-5-norbornène-2,3-dicarboximide avec du phosgène en présence d'amines tertiaires.

4. Utilisation de la N-chlorocarbonyloxy-5-norbornène-2,3-dicarboximide de formule I selon la revendication 1 pour la préparation:

- de carbonates asymétriques de formule IV:

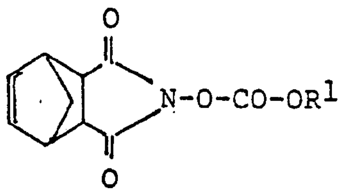 (IV)

dans laquelle

$R^1$ signifie t-butyle, 1-adamantyle, 2-diphénylpropyle, 9-méthylfluorényle, méthylsulfonyléthyle, benzyle ou autres radicaux courants en chimie des peptides,

- d'aminoacides ou peptides à uréthane protégé de formule V

$R^2$-NH-CO-$OR^1$ (V)

- dans laquelle

$R^1$ a la même signification que ci-dessus et

$R^2$ signifie un radical d'aminoacide ou de peptide,

- de supports activés de formule VII

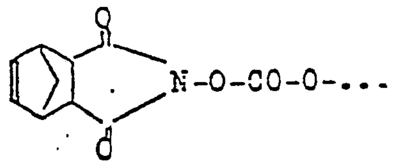 (VII)

et X

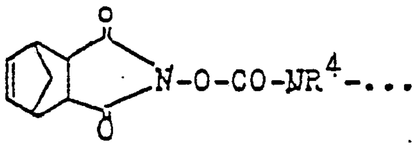 (X),

dans laquelle

$R^4$ signifie H, alcoyle, alcoyle substitué, aryle ou aryle substitué, de même que

- des complexes support-ligand de formules VIIIa, VIIIb, VIIIc, XIa, XIb et XIc:

$R^3$-NH-CO-O-... (VIIIa),

$R^3$-S-CO-O-... (VIIIb),

où $R^3$ signifie H, alcoyle, alcoyle substitué, aryle ou aryle substitué ou un radical d'aminoacide, de peptide ou de protéine,

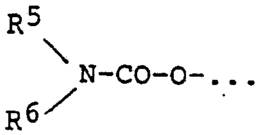 (VIIIc),

où $R^5$ et $R^6$ signifient H, alcoyle, alcoyle substitué, aryle ou aryle substitué,

$R^3$-NH-CO-$NR^4$-... (XIa),

$R^3$-S-CO-$NR^4$-... (XIb)

et

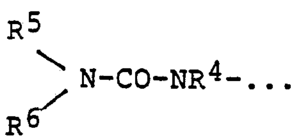 (XIc)

où

$R^3$, $R^4$, $R^5$ et $R^6$ ont la même signification que plus haut.